(19) Europäisches Patentamt
European Patent Office
Office européen des brevets

(11) Veröffentlichungsnummer: **0 361 185 B1**

(12) **EUROPÄISCHE PATENTSCHRIFT**

(45) Veröffentlichungstag der Patentschrift: **22.06.94**

(51) Int. Cl.[5]: **C07D 239/38**, C07D 239/58, A01N 43/54

(21) Anmeldenummer: **89116728.0**

(22) Anmeldetag: **09.09.89**

Die Akte enthält technische Angaben, die nach dem Eingang der Anmeldung eingereicht wurden und die nicht in dieser Patentschrift enthalten sind.

# (54) Substituierte 2-(Halogenmethylthio)-pyrimidin-Derivate.

(30) Priorität: **24.09.88 DE 3832530**
**13.04.89 DE 3912155**

(43) Veröffentlichungstag der Anmeldung:
**04.04.90 Patentblatt 90/14**

(45) Bekanntmachung des Hinweises auf die Patenterteilung:
**22.06.94 Patentblatt 94/25**

(84) Benannte Vertragsstaaten:
**BE CH DE ES FR GB IT LI NL**

(56) Entgegenhaltungen:
**EP-A- 0 033 195**
**DE-A- 3 510 178**
**US-A- 4 438 117**

**CHEMICAL ABSTRACTS, Band 83, Nr. 9, 1. September 1975, Seite 659,**

**Zusammenfassung Nr. 79188n, Columbus, Ohio, US; T.A. DASHEVSKAYA et al.:"Difluoromethylation of mercapto derivatives of pyrimidine",& UKR. KHIM. ZH. (Russ. Ed.) 1975, 41(5), 498-500**

(73) Patentinhaber: **BAYER AG**

**D-51368 Leverkusen(DE)**

(72) Erfinder: **Kraatz, Udo, Dr.**
**Andreasstrasse 22a**
**D-5090 Leverkusen 1(DE)**
Erfinder: **Kysela, Ernst, Dr.**
**Virchowstrasse 14**
**D-5060 Bergisch-Gladbach 1(DE)**
Erfinder: **Hartwig, Jürgen, Dr.**
**Franz-Esser-Strasse 44**
**D-5090 Leverkusen 3(DE)**
Erfinder: **Becker, Benedikt, Dr.**
**Metzkausener Strasse 14**
**D-4020 Mettmann(DE)**

**EP 0 361 185 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Die Erfindung betrifft neue substituierte 2-(Halogenmethylthio)-pyrimidin-Derivate, ein Verfahren zu ihrer Herstellung und ihre Verwendung als Schädlingsbekämpfungsmittel.

Es ist bereits bekannt, daß 2-Halogenalkylthio-substituierte Pyridine, wie beispielsweise 2-(Dibromfluormethylthio)-pyridin,insektizide Eigenschaften besitzen (vgl. DE-A 35 10 178).

Die Wirkung dieser Verbindungen ist jedoch, insbesondere bei niedrigen Aufwandmengen- und -konzentrationen, nicht immer in allen Anwendungsbereichen völlig zufriedenstellend.

Weiterhin sind 2-Halogenalkylthio-substituierte Pyrimidine, wie beispielsweise 2-(Chlormethylthio)-5-chlorpyrimidin und 2-(Iodmethylthio)-5-chlorpyrimidin, als Zwischenprodukte zur Herstellung von pharmazeutischen Produkten bekannt (vgl. EP-A 0 033 195).

Ferner sind 2-substituierte Thio-4,5-diarylpyrimidine, wie beispielsweise 2-(1,1,2,2-Tetrafluormethylthio)-4,5-bis(4-methoxyphenyl)-pyrimidin und 2-Trifluormethylthio-4,5-bis(4-fluorphenyl)-pyrimidin, bekannt. Diese Verbindungen haben u.a. eine starke entzündungshemmende und analgetische Wirkung (vgl. US-PS 4 438 117).

Darüber hinaus sind zahlreiche Synthesen für 2-Halogenalkylthio-substituierte Pyrimidin-Derivate bekannt (vgl. J. Heterocycl. Chem., 23, 1079-84; J. Heterocyl. Chem. 22, 1077-80; J. Chem. Soc., Perkin Trans., 1, 2499-503; Chem. Scr., 20, 11-13: Deposited Doc., VINITI 910, 5; Zh. Org. Khim., 15, 396-400; J. Org. Chem., 42, 3094-3096; Ukr. Khim. Zh. 42, 500-504; Ukr. Khim. Zh. 41, 498-500; Khim. Geterotsihl. Soedin., 1087-1088).

Biologische Wirkungen für diese Verbindungen werden nicht beschrieben.

Es wurden nun neue substituierte 2-(Halogenmethylthio)-pyrimidin-Derivate der Formel (I) gefunden,

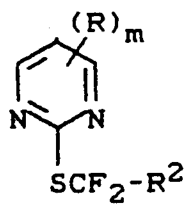

(I)

in welcher

R für Wasserstoff, jeweils geradkettiges oder verzweigtes Alkyl, Alkylthio, Alkoxy, Halogenalkyl, Halogenalkylthio, Halogenalkoxy mit jeweils 1 bis 4 Kohlenstoffatomen und im Fall von Halogenalkyl, Halogenalkylthio und Halogenalkoxy mit 1 bis 9 gleichen oder verschiedenen Halogenatomen, ferner für gegebenenfalls einfach oder mehrfach, gleich oder verschieden durch Halogen, Nitro, Alkyl oder Alkoxy mit jeweils 1 bis 4 Kohlenstoffatomen oder Halogenalkyl mit 1 bis 4 Kohlenstoffatomen und 1 bis 9 gleichen oder verschiedenen Halogenatomen substituiertes Phenyl sowie für Halogen steht,

$R^2$ für Wasserstoff, Chlor oder Brom steht und

m für eine Zahl 0, 1, 2 oder 3 steht,

ausgenommen die Verbindungen 2-(Difluormethylthio)-4,6-dimethyl-pyrimidin, 2,4-bis(Difluormethylthio)-6-methyl-pyrimidin und 4-(Difluormethoxy)-2-(difluormethylthio)-6-methyl-pyrimidin.

Man erhält die neuen substituierten 2-(Halogenmethylthio)-pyrimidin-Derivate der obigen Formel (I), wenn man 2-Mercaptopyrimidine der Formel (II),

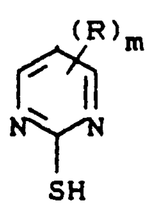

(II)

in welcher

R und der Index m die oben angegebene Bedeutung haben,

oder deren Salze

mit Alkylhalogeniden der Formel (III)

$R^2$-$CF_2$-X (III)

in welcher

$R^2$ die oben angegebene Bedeutung hat und

X für Halogen, vorzugsweise Chlor oder Brom steht,

gegebenenfalls in Gegenwart von Verdünnungsmitteln sowie gegebenenfalls in Gegenwart starker Basen alkyliert.

Weiterhin wurde gefunden, daß die neuen substituierten 2-(Halogenmethylthio)-pyrimidin-Derivate der obigen Formel (I) eine hohe Wirksamkeit gegen tierische Schädlinge, insbesondere gegen Insekten, Spinnentiere und Nematoden aufweisen. Sie eignen sich daher als Wirkstoffe in Schädlingsbekämpfungsmitteln, insbesondere als Insektizide, Akarizide und Nematizide.

Überraschenderweise zeigen die erfindungsgemäßen substituierten 2-(Halogenmethylthio)-pyrimidin-Derivate der Formel (I) eine bessere Wirkung als Schädlingsbekämpfungsmittel, insbesondere als Insektizide, Akarizide und Nematizide, als das aus dem Stand der Technik bekannte 2-(Dibrom-fluormethylthio)-pyridin.

Die erfindungsgemäßen substituierten 2-(Halogenmethylthio)-pyrimidin-Derivatestellen somit eine wertvolle Bereicherung der Technik dar.

Bevorzugt werden diejenigen Verbindungen der Formel (I), in welcher

R für Wasserstoff, Methyl, Ethyl, n- oder i-Propyl, n-, i-, s- oder t-Butyl, Methylthio, Methoxy, Halogenalkyl, Halogenalkylthio, Halogenalkoxy mit jeweils 1 bis 2 Kohlenstoffatomen und 1 bis 5 gleichen oder verschiedenen Halogenatomen; ferner für gegebenenfalls einfach bis dreifach, gleich oder verschieden durch Fluor, Chlor, Brom, Nitro, Methyl, Ethyl, Methoxy, Ethoxy oder Trifluormethyl substituiertes Phenyl sowie für Halogen steht,

$R^2$ für Wasserstoff, Chlor oder Brom steht und

m für eine Zahl 0, 1, 2 oder 3 steht,

ausgenommen die Verbindungen 2-(Difluormethylthio)-4,6-dimethyl-pyrimidin, 2,4-bis(Difluormethylthio)-6-methylpyrimidin und 4-(Difluormethoxy)-2-(difluormethylthio)-6-methyl-pyrimidin.

Verwendet man als Ausgangsstoffe beispielsweise 2-Mercaptopyrimidin und Dibromdifluormethan, so läßt sich der Reaktionsablauf des Herstellungsverfahrens durch das folgende Formelschema darstellen:

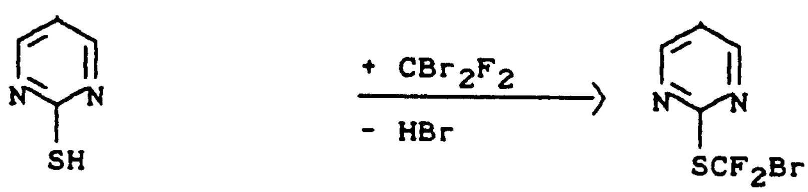


Die zur Durchführung des Herstellungsverfahrens als Ausgangsstoffe benötigten 2-Mercaptopyrimidine sind durch die Formel (II) allgemein definiert. In dieser Formel (II) stehen R und der Index m für diejenigen Bedeutungen, die oben bei der Beschreibung der neuen 2-(Halogenmethylthio)-pyrimidin-Derivate der Formel (I) für R und den Index m genannt wurden.

Die 2-Mercaptopyrimidine der Formel (II) sind bekannt und/oder können nach bekannten Verfahren in einfacher, analoger Weise hergestellt werden (vgl. Coll. Czech. Chem. Commun., 24, 1667 (1959); Chem. Ber. 104, 2975 (1971) und US-PS 4 438 117).

Die weiterhin zur Durchführung des Herstellungsverfahrens als Ausgangsstoffe benötigten Alkylhalogenide sind durch die Formel (III) allgemein definiert. In dieser Formel (III) haben $R^2$ und X diejenigen Bedeutungen, die oben bei der Beschreibung der neuen 2-(Halogenmethylthio)-pyrimidin-Derivate der Formel (I) für $R^2$ und X genannt wurden.

Die Verbindungen der Formel (III) sind allgemein bekannte Verbindungen der organischen Chemie.

Die Herstellung der neuen Verbindungen der Formel (I) erfolgt vorzugsweise unter Verwendung von Verdünnungsmitteln.

Als solche kommen dabei Wasser und praktisch alle für die Umsetzung üblichen inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Acetone, Methyl-ethyl-, Methyl-isopropyl- und Methyl-isobutyl-keton, Ester wie

Essigsäuremethylester und -ethylester, Nitrile wie Acetonitril und Propionitril, Alkohole wie Methanol, Ethanol, n-Propanol, Isopropanol und Butanol, Amide wie z.B. Dimethylformamid, Dimethylacetamid und N-Methyl-pyrrolidon sowie Dimethylsulfoxid, Tetramethylensulfon und Hexamethylphosphorsäuretriamid.

Als Basen, welche im Überschuß eingesetzt werden, kommen Alkalimetallhydroxide, wie z.B. Natrium- und Kaliumhydroxid, Erdalkalihydroxide, wie z.B. Calciumhydroxid, Alkalicarbonate und -alkoholate wie Natrium- und Kaliumcarbonat, Natrium- und Kaliummethylat bzw. -ethylat, Hydride wie zum Beispiel Natriumhydrid, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBU), 1,8-Diazabicyclo-[5,4,0]-undec-7-en (DBU) und 1,4-Diazabicyclo-[2,2,2]-octan (DABCO), infrage.

Die Reaktionstemperaturen können bei dem Verfahren zur Herstellung der neuen Verbindungen der Formel (I) in einem größeren Bereich variiert werden. Im allgemeinen arbeitet man bei Temperaturen zwischen 0 °C und 100 °C, vorzugsweise bei Temperaturen zwischen 10 °C und 70 °C.

Zur Durchführung des Herstellungsverfahrens der neuen Verbindungen der Formel (I) setzt man pro Mol 2-Mercaptopyrimidin der Formel (II) im allgemeinen 1,0 bis 10,0 Mol, vorzugsweise 1,0 bis 6,0 Mol an Alkylierungsmittel der Formel (III) und 1,0 bis 8,0 Mol, vorzugsweise 1,0 bis 5,0 Mol Base ein.

Das Verfahren zur Herstellung der neuen Verbindungen der Formel (I) wird im allgemeinen bei Normaldruck durchgeführt. Unter bestimmten Voraussetzungen kann jedoch auch unter erhöhtem oder vermindertem Druck gearbeitet werden.

Bei der Verwendung von gasförmigen Alkylierungsmitteln der Formel (III) können diese Verbindungen durch das Gemisch aus Verdünnungsmittel, Verbindung der Formel (II) und Base eingeleitet werden. Die Reaktionen werden im allgemeinen in einem geeigneten Verdünnungsmittel durchgeführt und das Reaktionsgemisch wird mehrere Stunden bei der jeweils erforderlichen Temperatur gerührt. Die Aufarbeitung und Isolierung der Reaktionsprodukte der Formel (I) erfolgt in allgemein üblicher Art und Weise.

Die Herstellung und die Verwendung der erfindungsgemäßen Wirkstoffe geht aus den nachfolgenden Beispielen hervor.

Herstellungsbeispiele:

Beispiel 1

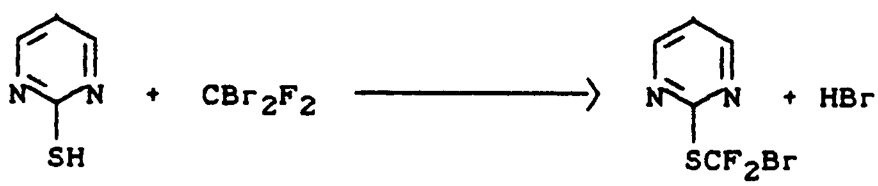

Zu einer Lösung von 11,2 g (0,1 Mol) 2-Mercaptopyrimidin in 200 ml Dimethylformamid werden bei 20-30 °C unter Rühren portionsweise 7,5 g (0,25 Mol) Natriumhydrid (80 %ige Öl-Suspension) zugesetzt. Nach 30 Minuten Rühren bei 20 °C fügt man 96 g (0,45 Mol) Dibromdifluormethan zu und läßt über Nacht ebenfalls bei 20 °C weiterrühren. Das Dimethylformamid wird bei 20-30 °C unter vermindertem Druck entfernt, der Rückstand mit Methylenchlorid/Wasser behandelt und die organische Phase abgetrennt. Nach dem Trocknen über Magnesiumsulfat wird unter vermindertem Druck das Lösungsmittel entfernt und der Rückstand destilliert.

Man erhält 15,2 g (25 % der Theorie) 2-(Bromdifluormethylthio)-pyrimidin mit dem Siedepunkt Kp 100 °C/0,3 mm. 0,3 mm.

Beispiel 2

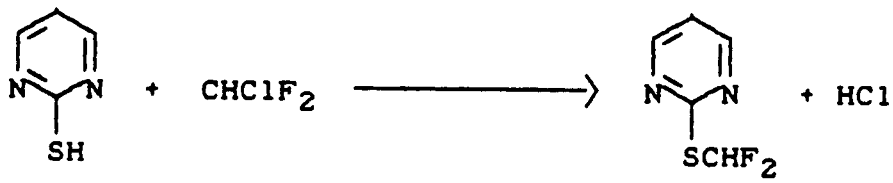

In eine Lösung von 25 g (0,22 Mol) 2-Mercaptopyrimidin in 120 ml Isopropanol und 30 g (0,73 Mol) Natriumhydroxid in 45 ml Wasser leitet man unter Rühren bei 40-50 °C 100 g (1,2 Mol) Chlordifluormethan ein. Die Reaktion ist schwach exotherm. Nach Beendigung der Reaktion verdünnt man mit etwa 400 ml Wasser und extrahiert das Produkt mit Methylenchlorid. Die organische Phase wird unter vermidertem Druck eingeengt und der Rückstand unter vermidertem Druck destilliert.

Man erhält 17,7 g (49,7 % der Theorie) 2-(Difluormethylthio)-pyrimidin mit dem Siedepunkt Kp 95 °C/ 20 mm.

Analog Beispiel 1 und 2 bzw. dem erfindungsgemäßen Verfahren können die in der nachfolgenden Tabelle 1 angegebenen Verbindungen der Formel (I) hergestellt werden:

Tabelle 1

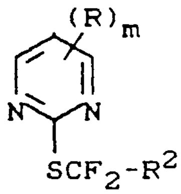

(I)

| Bsp. Nr. | 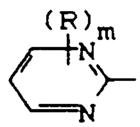 | $R^2$ | physikalische Konstante |
|---|---|---|---|
| 3 | 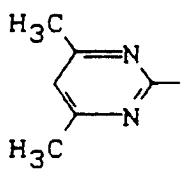 | -Br | $n_D^{20}$ : 1,5255 |
| 4 | 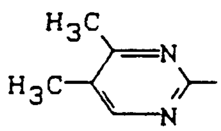 | -Br | $n_D^{24}$ : 1,5168 |
| 5 | 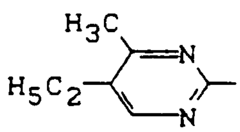 | -Br | $n_D^{24}$ : 1,5055 |
| 6 | 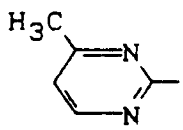 | -Br | $n_D^{22}$ : 1,5230 |
| 7 | 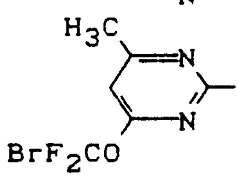 | -Br | $n_D^{20}$ : 1,4700 |
| 8 | 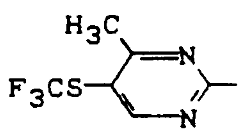 | -Br | $n_D^{22}$ : 1,4970 |
| 9 | 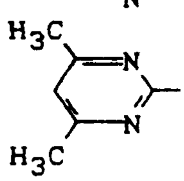 | -H | $n_D^{23}$ : 1,4994 |

Tabelle 1 - Fortsetzung

| Bsp. Nr. | 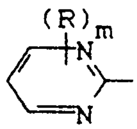 | $R^2$ | physikalische Konstante |
|---|---|---|---|
| 10 | 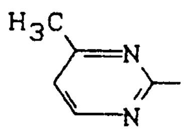 | -H | $n_D^{23}$ : 1,5034 |
| 11 | 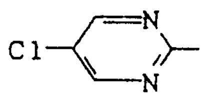 | -H | Kp 45° C/ 0,1 mbar |
| 12 | 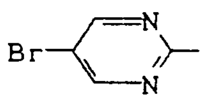 | -H | Fp: 48° C |
| 13 | 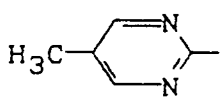 | -H | Fp: 47° C |
| 14 | 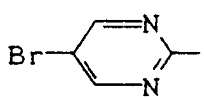 | -Br | Fp: 80° C |
| 15 | 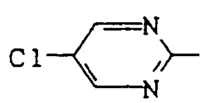 | -Br | $n_D^{23}$ : 1,5420 |
| 16 | 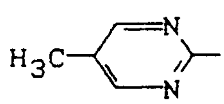 | -Br | $n_D^{23}$ : 1,5258 |

Tabelle 1 - Fortsetzung

| Bsp. Nr. | 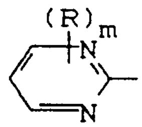 | $R^2$ | physikalische Konstante |
|---|---|---|---|
| 17 | 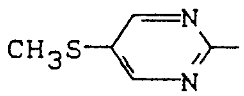 | -H | Fp: 42° C |
| 18 | 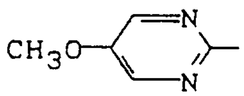 | -Br | Fp: 40° C |
| 19 | 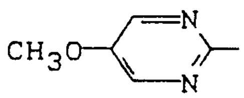 | -H | Fp: 40° C |

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci,

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima,

Tortrix viridana.

Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die erfindungsgemäßen Wirkstoffe der Formel (I) zeichnen sich durch eine hervorragende insektizide Wirksamkeit aus. So ist insbesondere die hervorragende Wirkung gegen Nematoden, wie z.B. Globodera rostochiensis und Meloidogyne incognita zu nennen sowie die ausgezeichnete Wirkung gegen Bodeninsekten, wie z.B. Diabrotica balteata-Larven.

Darüberhinaus zeigen die erfindungsgemäßen Wirkstoffe auch eine hervorragende Wirkung gegen pflanzenschädigende Insekten, wie z.B. Tetranychus-Arten und Plutella-Raupen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte al phatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und

Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die biologische Wirksamkeit der erfindungsgemäß zu verwendenden Verbindungen soll anhand der folgenden Beispiele erläutert werden.

Verwendungsbeispiele

In den nachfolgenden Verwendungsbeispielen wird die nachstehend angegebene Verbindung als Vergleichssubstanz eingesetzt:

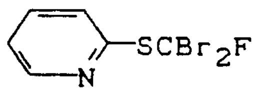

(A)

2-(Dibrom-fluormethylthio)-pyridin.

Die Verbindung (A) ist bekannt aus DE-A 35 10 178.

Beispiel A

Grenzkonzentrations-Test / Nematoden

| | |
|---|---|
| Testnematode: | Meloidogyne incognita |
| Lösungsmittel: | 3 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27 °C.

Nach vier Wochen werden die Salatwurzeln auf Nematodenbefall (Wurzelgallen) untersucht und der

Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigen z.B. die folgenden Verbindungen der Herstellungsbeispiele 1, 3, 4, 5, 6, 13, 14, 15 und 16 überlegene Wirksamkeit gegenüber dem Stand der Technik:

Tabelle A

Nematizide
*Meloidogyne incognita*

| Wirkstoff | Abtötungsgrad in % bei Wirkstoffkonzentration in ppm |
|---|---|
| 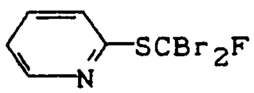 (bekannt) (A) | 20 ppm = 0 % |
| 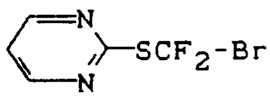 (1) | 20 ppm = 100 % |
| 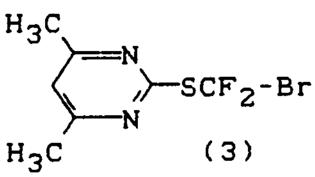 (3) | 20 ppm = 95 % |
| 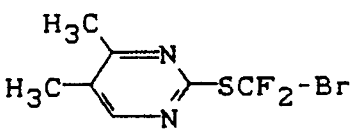 (4) | 20 ppm = 100 % |
| 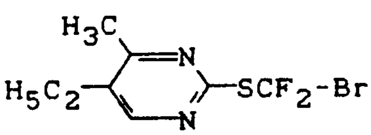 (5) | 20 ppm = 95 % |
| 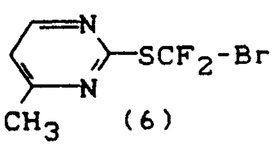 (6) | 20 ppm = 95 % |

Tabelle A (Fortsetzung)

Nematizide

Meloidogyne incognita

| Wirkstoff | Abtötungsgrad in % bei Wirkstoffkonzentration in ppm |
|---|---|
| 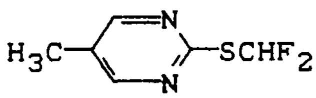 (13) | 20 ppm = 0 % |
| 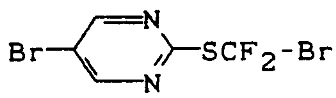 (14) | 20 ppm = 100 % |
| 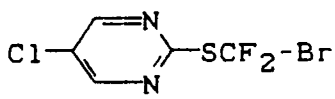 (15) | 20 ppm = 100 % |
| 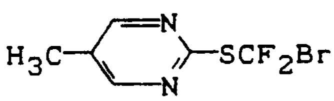 (16) | 20 ppm = 100 % |

Beispiel B

Grenzkonzentrations-Test

Testnematode: Globodera rostochiensis
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, pflanzt Kartoffeln ein und hält die Töpfe bei einer Gewächshaus-Temperatur von 18 °C.

Nach sechs Wochen werden die Kartoffelwurzeln auf Zysten untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigen z.B. die folgenden Verbindungen der Herstellungsbeispiele 1, 4, 5, 6, 14 und 15 überlegene Wirksamkeit gegenüber dem Stand der Technik:

## Tabelle B

Nematizide
Globodera rostochiensis

| Wirkstoff | Abtötungsgrad in % bei Wirkstoffkonzentration in ppm |
|---|---|
| 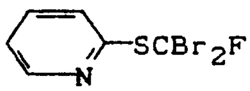 (bekannt) (A) | 20 ppm = 0 % |
| 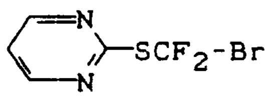 (1) | 20 ppm = 100 % |
| 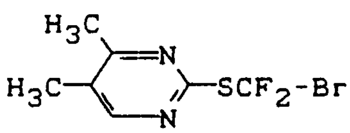 (4) | 20 ppm = 100 % |
| 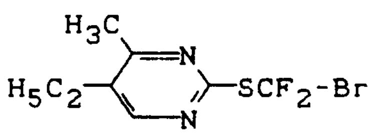 (5) | 20 ppm = 100 % |
| 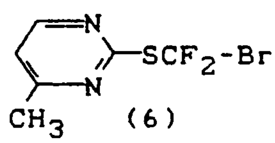 (6) | 20 ppm = 95 % |
| 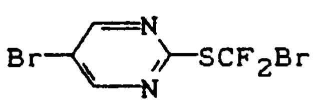 (14) | 20 ppm = 100 % |
| 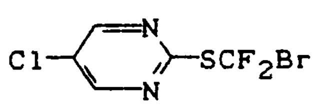 (15) | 20 ppm = 100 % |

Beispiel C

Tetranychus-Test (resistent)

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigt z.B. die folgende Verbindung des Herstellungsbeispiels 4 überlegene Wirksamkeit gegenüber dem Stand der Technik.

## Tabelle C

(pflanzenschädigende Milben)
Tetranychus (resistent)

| Wirkstoff | Wirkstoffkonzentration in % | Abtötungsgrad in % nach 7d |
|---|---|---|
| 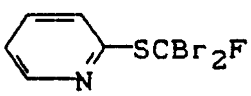 (bekannt) (A) | 0,1 | 0 |
| 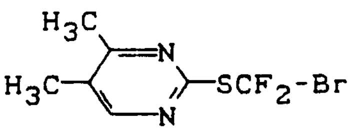 (4) | 0,1 | 95 |

Beispiel D

Plutella-Test

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele 1, 4 und 5 überlegene Wirksamkeit gegenüber dem Stand der Technik:

## Tabelle D

(pflanzenschädigende Insekten)
Plutella-Test

| Wirkstoff | Wirkstoffkonzentration in % | Abtötungsgrad in % nach 3 d |
|---|---|---|
| 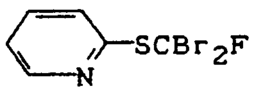 (bekannt) (A) | 0,1 | 0 |
| 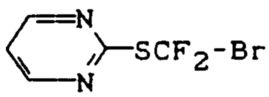 (1) | 0,1 | 100 |
| 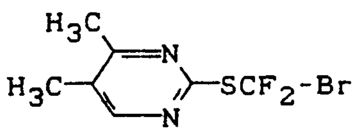 (4) | 0,1 | 100 |
| 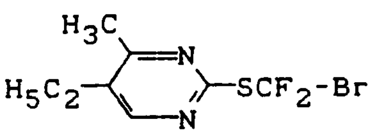 (5) | 0,1 | 100 |

Beispiel E

Grenzkonzentrations-Test / Bodeninsekten

Testinsekt: Diabrotica balteata - Larven im Boden
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in 0,5 l Töpfe und läßt diese bei 20 °C stehen.

Sofort nach dem Ansatz werden je Topf 6 vorgekeimte Maiskörner gelegt. Nach 2 Tagen werden in den behandelten Boden die entsprechenden Testinsekten gesetzt. Nach weiteren 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele 2, 7, 8, 10, 14 und 15 überlegene Wirksamkeit gegenüber dem Stand der Technik.

## Tabelle E

Bodeninsektizide
Diabrotica balteata-Larven im Boden

| Wirkstoff | Abtötungsgrad in % bei Wirkstoffkonzentration in ppm |
| --- | --- |
| 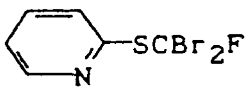 (bekannt) (A) | 20 ppm = 0 % |
| 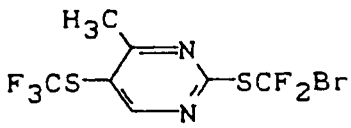 (8) | 20 ppm = 100 % |
| 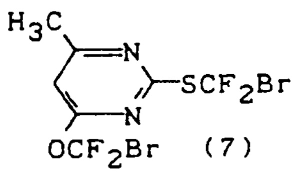 (7) | 20 ppm = 100 % |
| 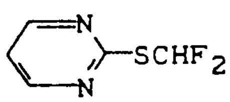 (2) | 20 ppm = 100 % |
| 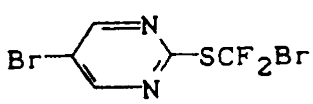 (14) | 20 ppm = 100 % |
| 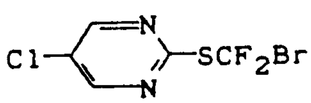 (15) | 20 ppm = 100 % |

**Patentansprüche**

1. Substituierte 2-(Halogenmethylthio)-pyrimidin-Derivate der Formel (I)

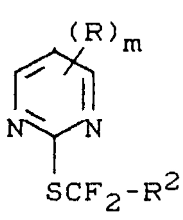

(I)

in welcher

R für Wasserstoff, jeweils geradkettiges oder verzweigtes Alkyl, Alkylthio, Alkoxy, Halogenalkyl, Halogenalkylthio, Halogenalkoxy mit jeweils 1 bis 4 Kohlenstoffatomen und im Fall von Halogenalkyl, Halogenalkylthio und Halogenalkoxy mit 1 bis 9 gleichen oder verschiedenen Halogenatomen, ferner für gegebenenfalls einfach oder mehrfach, gleich oder verschieden durch Halogen, Nitro, Alkyl oder Alkoxy mit jeweils 1 bis 4 Kohlenstoffatomen oder Halogenalkyl mit 1 bis 4 Kohlenstoffatomen und 1 bis 9 gleichen oder verschiedenen Halogenatomen substituiertes Phenyl sowie für Halogen steht,

$R^2$ für Wasserstoff, Chlor oder Brom steht und

m für eine Zahl 0, 1, 2 oder 3 steht,

ausgenommen die Verbindungen 2-(Difluormethylthio)-4,6-dimethyl-pyrimidin, 2,4-bis(Difluormethylthio)-6-methyl-pyrimidin und 4-(Difluormethoxy)-2-(difluormethylthio)-6-methyl-pyrimidin.

**2.** Substituierte 2-(Halogenmethylthio)-pyrimidin-Derivate der Formel (I) gemäß Anspruch 1, in welcher

R für Wasserstoff, Methyl, Ethyl, n- oder i-Propyl, n-, i-, s- oder t-Butyl, Methylthio, Methoxy, Halogenalkyl, Halogenalkylthio, Halogenalkoxy mit jeweils 1 bis 2 Kohlenstoffatomen und 1 bis 5 gleichen oder verschiedenen Halogenatomen; ferner für gegebenenfalls einfach bis dreifach, gleich oder verschieden durch Fluor, Chlor, Brom, Nitro, Methyl, Ethyl, Methoxy, Ethoxy oder Trifluormethyl substituiertes Phenyl sowie für Halogen steht,

$R^2$ für Wasserstoff, Chlor oder Brom steht und

m für eine Zahl 0, 1, 2 oder 3 steht,

ausgenommen die Verbindungen 2-(Difluormethylthio)-4,6-dimethyl-pyrimidin, 2,4-bis(Difluormethylthio)-6-methyl-pyrimidin und 4-(Difluormethoxy)-2-(difluormethylthio)-6-methyl-pyrimidin.

**3.** Verfahren zur Herstellung von substituierten 2-(Halogenmethylthio)-pyrimidin-Derivaten der Formel (I) gemäß Anspruch 1, dadurch gekennzeichnet, daß man 2-Mercaptopyrimidine der Formel (II),

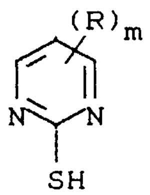

(II)

in welcher

R und der Index m die oben angegebene Bedeutung haben,

oder deren Salze

mit Alkylhalogeniden der Formel (III),

$R^2$-$CF_2$-X (III)

in welcher

$R^2$ die oben angegebene Bedeutung hat und

X für Halogen steht,

gegebenenfalls in Gegenwart von Verdünnungsmitteln sowie gegebenenfalls in Gegenwart starker Basen alkyliert.

**4.** Schädlingsbekämpfungsmittel, gekennzeichnet durch einen Gehalt an mindestens einem substituierten 2-(Halogenmethylthio)-pyrimidin-Derivat der Formel (I) gemäß Anspruch 1.

**5.** Verfahren zur Bekämpfung von tierischen Schädlingen, insbesondere von Insekten, Spinnentieren und Nematoden, dadurch gekennzeichnet, daß man substituierte 2-(Halogenmethylthio)-pyrimidin-Derivate der Formel (I) gemäß Anspruch 1 auf tierische Schädlinge und/oder ihren Lebensraum einwirken läßt.

**6.** Verwendung von substituierten 2-(Halogenmethylthio)-pyrimidin-Derivaten der Formel (I) gemäß Anspruch 1 zur Bekämpfung von tierischen Schädlingen.

7. Verfahren zur Herstellung von Schädlingsbekämpfungsmitteln, dadurch gekennzeichnet, daß man substituierte 2-(Halogenmethylthio)-pyrimidin-Derivate der Formel (I) gemäß Anspruch 1 mit Streckmitteln und/oder oberflächenaktiven Mitteln vermischt.

## Claims

1. Substituted 2-(halogenomethylthio)-pyrimidine derivatives of the formula (I)

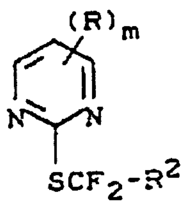

(I)

in which

R represents hydrogen, in each case straight-chain or branched alkyl, alkylthio, alkoxy, halogenoalkyl, halogenoalkylthio, halogenoalkoxy, each of which has 1 to 4 carbon atoms and, in the case of halogenoalkyl, halogenoalkylthio and halogenoalkoxy, 1 to 9 identical or different halogen atoms, furthermore represents phenyl which is optionally monosubstituted or polysubstituted by identical or different substituents from the series comprising halogen, nitro, alkyl or alkoxy, each of which has 1 to 4 carbon atoms, or halogenoalkyl having 1 to 4 carbon atoms and 1 to 9 identical or different halogen atoms, or represents halogen,

$R^2$ represents hydrogen, chlorine or bromine and

m represents a number 0, 1, 2 or 3,

with the exception of the compounds 2-(difluoromethylthio)-4,6-dimethylpyrimidine, 2,4-bis-(difluoromethylthio)-6-methylpyrimidine and 4-(difluoromethoxy)-2-(difluoromethylthio)-6-methylpyrimidine.

2. Substituted 2-(halogenomethylthio)-pyrimidine derivatives of the formula (I) according to Claim 1, in which

R represents hydrogen, methyl, ethyl, n- or i-propyl, n-, i-, s- or t-butyl, methylthio, methoxy, halogenoalkyl, halogenoalkylthio, halogenoalkoxy, each of which has 1 to 2 carbon atoms and 1 to 5 identical or different halogen atoms; furthermore represents phenyl which is optionally monosubstituted to trisubstituted by identical or different substituents from the series comprising fluorine, chlorine, bromine, nitro, methyl, ethyl, methoxy, ethoxy or trifluoromethyl, or represents halogen,

$R^2$ represents hydrogen, chlorine or bromine and

m represents a number 0, 1, 2 or 3,

with the exception of the compounds 2-(difluoromethylthio)-4,6-dimethylpyrimidine, 2,4-bis(difluoromethylthio)-6-methylpyrimidine and 4-(difluoromethoxy)-2-(difluoromethylthio)-6-methylpyrimidine.

3. Process for the preparation of substituted 2-(halogenomethylthio)-pyrimidine derivatives of the formula (I) according to Claim 1, characterized in that 2-mercaptopyrimidines of the formula (II)

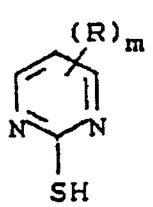

(II)

in which

R and the index m are as defined above,

or salts thereof

are alkylated with alkyl halides of the formula (III)

$R^2$-$CF_2$-X (III)

in which

$R^2$ has the abovementioned meaning and

X represents halogen,

if appropriate in the presence of diluents and if appropriate in the presence of strong bases.

**4.** Pesticides, characterized in that they contain at least one substituted 2-(halogenomethylthio)-pyrimidine derivative of the formula (I) according to Claim 1.

**5.** Method of combating animal pests, in particular insects, arachnids and nematodes, characterized in that substituted 2-(halogenomethylthio)-pyrimidine derivatives of the formula (I) according to Claim 1 are allowed to act on animal pests and/or their environment.

**6.** Use of substituted 2-(halogenomethylthio)-pyrimidine derivatives of the formula (I) according to Claim 1 for combating animal pests.

**7.** Process for the preparation of pesticides, characterized in that substituted 2-(halogenomethylthio)-pyrimidine derivatives of the formula (I) according to Claim 1 are mixed with extenders and/or surfactants.

**Revendications**

**1.** Dérivés substitués de 2-(halogénométhylthio)-pyrimidine de formule (I)

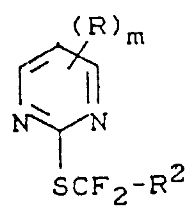

(I)

dans laquelle

R représente l'hydrogène, un groupe, linéaire ou ramifié alkyle, alkylthio, alcoxy, halogénoalkyle, halogénoalkylthio, halogénoalcoxy de 1 à 4 atomes de carbone et, dans le cas d'un groupe halogénoalkyle, halogénoalkylthio et halogénoalcoxy, comportant 1 à 9 atomes d'halogène identiques ou différents, un groupe phényle éventuellement substitué une ou plusieurs fois, de manière identique ou différente, par halogène, nitro, alkyle ou alcoxy ayant 1 à 4 atomes de carbone ou par halogénoalkyle ayant 1 à 4 atomes de carbone et 1 à 9 atomes d'halogène identiques ou différents, ainsi qu'un halogène,

$R^2$ représente l'hydrogène, le chlore ou le brome et

m représente un nombre égal à 0, 1, 2 ou 3,

à l'exception des composés 2-(difluorométhylthio)-4,6-diméthylpyrimidine, 2,4-bis(difluorométhylthio)-6-méthylpyrimidine et 4-(difluorométhoxy)-2-(difluorométhylthio)-6-méthylpyrimidine.

**2.** Dérivés substitués de 2-(halogénométhylthio)-pyrimidine de formule (I) selon la revendication 1, dans lesquels

R représente l'hydrogène, un groupe méthyle, éthyle, n- ou i-propyle, n-, i-, s- ou t-butyle, méthylthio, méthoxy, halogénoalkyle, halogénoalkylthio, halogénoalcoxy de 1 à 2 atomes de carbone et de 1 à 5 atomes d'halogène identiques ou différents, un groupe phényle éventuellement substitué une à trois fois, de manière identique ou différente, par fluor, chlore, brome, nitro, méthyle, éthyle, méthoxy, éthoxy ou trifluorométhyle, et un halogène,

$R^2$ représente l'hydrogène, le chlore ou le brome et

m représente un nombre égal à 0, 1, 2 ou 3,

à l'exception des composés 2-(difluorométhylthio)-4,6-diméthylpyrimidine, 2,4-bis(difluorométhylthio)-6-méthylpyrimidine et 4-(difluorométhoxy-2-(difluorométhylthio)-6-méthylpyrimidine.

3. Procédé de préparation de dérivés substitués de 2-(halogénométhylthio)-pyrimidine de formule (I) selon la revendication 1, caractérisé en ce que l'on alkyle des 2-mercaptopyrimidines de formule (II)

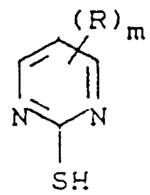

(II)

dans laquelle
R et l'indice m ont la signification indiquée ci-dessus,
ou leurs sels
avec des halogénures d'alkyle de formule (III)

$R^2$-$CF_2$-X (III)

dans laquelle
$R^2$ a la signification indiquée ci-dessus et
X représente un halogène,
éventuellement en présence de diluants et éventuellement en présence de bases fortes.

4. Agents de lutte contre les nuisibles, caractérisés par une teneur en au moins un dérivé substitué de 2-(halogénométhylthio)-pyrimidine de formule (I) selon la revendication 1.

5. Procédé de lutte contre les animaux nuisibles, en particulier contre les insectes, les arachnides et les nématodes, caractérisé en ce que l'on fait agir des dérivés substitués de 2-(halogénométhylthio)-pyrimidine de formule (I) selon la revendication 1 sur les animaux nuisibles et/ou leur biotope.

6. Utilisation de dérivés substitués de 2-(halogénométhylthio)-pyrimidine de formule (I) selon la revendication 1 pour la lutte contre les animaux nuisibles.

7. Procédé de préparation d'agents de lutte contre les nuisibles, caractérisé en ce que l'on mélange des dérivés substitués de 2-(halogénométhylthio)-pyrimidine de formule (I) selon la revendication 1 avec des diluants et/ou des agents tensioactifs.